# EUROPEAN PATENT APPLICATION

(11) **EP 1 358 858 A1**
(43) Date of publication of application: **05.11.2003**
(21) Application number: 03252769.9
(22) Date of filing: 01.05.2003
(51) Int. Cl.: A61F 2/16

(54) **Anterior chamber phakic lens**

(30) Priority: 04.05.2002 US 139836
(71) Applicant: Buzard, Kurt, Las Vegas, Nevada 89117 (US)
(72) Inventor: Buzard, Kurt, Las Vegas, Nevada 89117 (US)
(74) Representative: Franks, Robert Benjamin

(57) **Abstract**

A unique anterior chamber phakic lens (30) for implantation is provided. The foldable optical zone portion (36) of the lens is secured to a pair of diametrically opposed haptic members (32,34) extending outwardly from the optical zone periphery to the angle of the eye. Each of the haptic members comprises an elongated arm portion (32a,34a) and a distal arcuate portion (32b,34b) with an inward curvature having an abraded surface (32c,34c). The inventive phakic lens is angle fixated by inducing a slight amount of scar tissue where the distal ends of lens haptics rest in the angle. Accordingly, the unique phakic lens minimizes lens related etiology such as iris chafing, glaucoma, uveitis, distortion of the iris, and/or corneal decompensation.

## Description

### BACKGROUND OF THE INVENTION

The present invention provides an improved angle fixated phakic lens and small incision surgical methods for implantation.

Presbyopia is a normal aging problem in which we gradually lose the ability to read without visual aid, starting at around age 40.

The origins of refractive surgery can be traced back nearly a century ago to the pioneering efforts of Lans for the correction of astigmatism. It has only been in the past 20 years though, that there has been an explosion of interest in operations designed to reduce or eliminate the need for glasses. The instant invention is the result of the modern renaissance of refractive surgery with many contributions to the treatment of astigmatism, nearsightedness and centration.

In the eye, two elements participate in the bending light to allow clear vision: the cornea and the human lens. The cornea is most often the initial choice to change refraction because of its convenient location on the surface of the eye. The relative ease of corneal surgery has lead refractive surgeons to develop numerous procedures to change the shape and curvature of the cornea, which accounts for about two-thirds of the light bending power in the eye.

The natural lens, located on the inside of the eye, is the remaining location to alter the bending of light. It accounts for the other one-third of the eyes' refractive power. The correction of the natural lens with an artificial lens is as natural a method to change the light bending power of the eye as corneal refractive operations. Inadequate natural lens power can be remedied by implantation with an intraocular lens of proper power and/or changing the shape or curvature of the cornea with corneal refractive surgeries such as LASIK.

In LASIK, corneal tissue is removed to achieve refractive changes in hopes of eliminating the need for glasses, but the cornea is a relatively thin tissue--only 0.6 mm thick. The severity of nearsightedness and farsightedness that can be corrected with corneal refractive surgery is limited by the amount of tissue necessary to maintain corneal strength. In general, more than 15 diopters of refractive correction (a severe degree of nearsightedness) cannot be corrected with the LASIK operation alone.

In this respect, refractive surgeons have realized that corneal refractive surgery (especially LASIK) cannot correct high degrees of ametropia without adversely changing the aspheric contour of the cornea. This fact has led such surgeons to a renewed interest in phakic intraocular lenses.

Phakic intraocular lenses can be combined with LASIK for extreme myopia in a procedure called Bioptics. Other advantages of phakic IOLs include very rapid acuity recovery.

Presently, intraocular lenses have gained wide acceptance in the refractive correction of and in the replacement of human crystalline lens after a variety of surgical procedures.

Of particular interest in the context of the present invention is the development of surgical techniques requiring relatively small incisions in the ocular tissue as disclosed in U.S. Patent No. 4,002,169 and U.S. Patent No. 3,996,935. A number of skilled artisans have also disclosed intraocular lens structures comprising an optical zone portion of foldable materials such as acrylic or silicone suitable for optical use such as U.S. Patent No. 4,573,998.

Since Ridley implanted the first artificial lens in about 1949, the problems associated with lens implantation have received a great deal of attention from ophthalmic surgeons. Various types of artificial lenses have been proposed, and appropriate surgical procedures have been developed which strive to achieve clear vision while reducing patient discomfort and reducing post-operative complications. Reference is made in this connection to Pseudophakos by J. Jaffe, et al.; "History of Intraocular Implants" by D.P. Choyce (Annals of Ophthalmology, October 1973); U.S. Patent No. 3,991,426 issued to Flom on November 16, 1976; U.S. Patent No. 4,092,743 issued to Kelman on November 8, 1977; U.S. Patent No. 4,446,581 issued to Blake on May 8, 1984; U.S. Patent No. 4,468,820 issued to Uhler, et al. on September 4, 1984; U.S. Patent No. 4,568,347 issued to Reichert on February 4, 1986; U.S. Patent No. 4,673,406 issued to Schiegel on June 16, 1987; U.S. Patent No. 5,192,319 issued to Worst on March 9, 1993; U.S. Patent No. 5,571,177 issued to Deacon, et al. on November 5, 1996; U.S. Patent No. 5,928,282 issued to Nigam on July 27, 1999; U.S. Patent No. 6,045,578 issued to Collins, et al. on April 4, 2000; U.S. Patent No. 6,051,024 issued to Cumming on April 18, 2000; U.S. Patent No. 6,110,202 issued to Barraquer on August 29, 2000; U.S. Patent No. 6,129,760 issued to Fedorov on October 10, 2000; and U.S. Patent No. 6,152,958 issued to Nordan on November 28, 2000; which disclosures are hereby incorporated by this reference.

Intraocular lenses placed in the front of the iris, are generally referred to as "anterior chamber lenses." The Choyce lens and Kelman lens were examples of these rigid anterior chamber lenses which were known in some cases, however, to cause a syndrome called UGH, resulting in distortion of the iris, failure of the cornea and possible loss of the eye.

Thus, UGH syndrome (the triad of uveitis, glaucoma, and hyphema) has been a deterrent to continued use of such anterior chamber intraocular lenses.

More recently, new materials, new designs and new manufacturing as well as polishing techniques have improved the quality of the phakic intraocular lenses, thereby reducing complication rates.

Current anterior chamber IOLS include the Baikoff ZMB, with a 4-mm optic, and the Nuvita MA20 lens, with a 4.5-mm optic, which has a larger optical zone and "kinder feet for the angle." A modification of the Nuvita lens is the Morcher ZSAL-4, which has a slightly larger optical size with an optic of 5 mm. The No Touch IOL also has a 4-mm diameter.

Iris-fixated lenses include the Worst-Fechner Iris Claw IOL, the Artisan IOL, and the Krumeich Active Closure Iris Lens. One of the more popular lenses was the Binkhorst lens, which had clips that fixated the lens on the iris. However, if the pupil dilated the lens would fall to the back of the eye. Additionally, such iris clip lenses, had a tendency to destroy the iris.

The Shearing J loop posterior chamber lens was a major improvement in lens design including flexible "haptics" which allowed the lens to adapt to different sizes of eyes. The basic design of this widely accepted lens, was a loop of prolene or other flexible material extending out between 11 and 14 mm from a central round disc of hard plastic between 5 and 6 mm. The plastic lens materials varied but the basic design of the Shearing lens remained the same and implantation of this lens into the bag of the cataract was a fundamental reason that "phacoemulsification" gained such importance among practicing ophthalmic surgeons.

Current posterior IOLs include the Fyodorov Silicone IOL, a Staar ICL, made of 0.1% porcine collagen, and the IVI Medennium, made of 100% silicone.

A number of original attempts to correct high myopia with phakic IOLs failed due to poor technology, lack of viscoelastics, poor lens design (very large and heavy), and lack of knowledge of anterior chamber anatomy.

In this regard, the anatomy of the angle and/or sulcus is essential to understanding the pathophysiologic changes. Compression of these structures limits blood flow, encourages fibrosis and inflammation.

Uveitis is an inflammation of the uveal tract which consists of the choroid, the ciliary body, and the iris. It may be classified as anterior, intermediate (pars planitis), or posterior (choroiditis) uveitis. Anterior uveitis involves the anterior portion of the uvea (i.e., the iris and ciliary body). "Ititis" refers to an inflammation of the iris only, while "iridocyclitis" involves both the iris and the ciliary body. However, the terms anterior uveitis, iritis, and iridocyclitis are often used synonymously. Anterior uveitis is an intraocular inflammation of the uveal structures anterior to the middle of the vitreous cavity. Along with conjunctivitis, keratitis, and acute glaucoma, it is one of a group of ocular conditions commonly termed "red-eye."

It is difficult to properly size anterior chamber phakic lenses without visualization of the internal structures. Even with careful sizing and attention to finishing of the footplates, the pressure applied to the angle by such conventional phakic lenses is excessive with rubbing and with time the footplates will usually erode, making the fit worse.

Accordingly, those skill in the art have recognized a significant need for an improved phakic IOL and attendant method for refractive correction of the eye that provides predictability, safety and adjustability. The present invention fulfills these needs.

### SUMMARY OF THE INVENTION

The present invention provides unique anterior chamber phakic lens structures and methods for implantation. The foldable optical zone portion of the lens is secured to a pair of diametrically opposed flexible haptic members extending longitudinally from the optical zone. Each of the haptic members comprises an elongated arm portion and a distal arcuate portion with an inward curvature. The outer surface of the distal arcuate portion is roughened, such as by sanding. When placed in the anterior chamber the abraded distal end of the haptic rests in the angle of the eye and held by induced mild fibrosis of the surrounding ocular tissue.

In one embodied form, the anterior chamber phakic lens comprises:
(a) a foldable plano convex or plano concave optical zone portion having an optical axis, wherein the plano surface faces the natural lens after placement in the eye; and
(b) a pair of diametrically opposed flexible haptic members extending outwardly from the periphery of the optical zone portion to the angle of the eye. Each of the haptic members includes an elongated arm portion spaced in parallel relation to a median line through said optical zone portion, and an distal arcuate portion with an inward curvature terminating at the median line through the optical zone portion. The distal arcuate portion of the haptic has a rough exterior surface and is sized to extend from about 1 to 4 millimeter from the interior wall of the angle.
   The unique lens is flat, neither vaulted up or down, having a plano convex or plano concave cross-section, with the plano section of the lens placed toward the natural lens thus avoiding touching the natural lens. The lens haptics are relatively short in length with respect to the size of the optic and when surgically placed extend just to the angle and have no return. The distal portion of the haptic that rests in the angle is slightly abraded to induce a mild fibrosis which assists lens fixation.
   The inventive method for implantation of the unique phakic lens for refractive correction of an eye, comprises the steps of: (a) providing a phakic lens having a foldable plano surfaced optical zone portion secured to a pair of diametrically opposed haptic members extending outwardly from the periphery of the optical zone portion to the angle of the eye, the distal portion of such haptic members having a roughened surface; (b) deforming the phakic lens by folding the optical zone portion to a diameter of about 80% or less of the cross-sectional diameter of the optic in an unstressed state; (c) inserting the intraocular lens through a relatively small incision made in the ocular tissue for placement in the anterior chamber of the eye;
(d) allowing the optical zone portion of the lens to return to its original configuration, full size and fixed focal length wherein the plano surface of the lens faces the natural lens of the eye; and (e) effecting placement of the lens haptics to rest in the angle after insertion in the eye whereby the phakic lens haptics create light attachments in the angle with minimal pressure on the blood vessels in ocular tissue.

Accordingly, the phakic lens of the present invention is held in place by the creation of mild fibrosis where the lens haptic rests in the angle with minimal compression.

The unique phakic lens and implantation methods minimize lens related etiology such as iris chafing, glaucoma, uveitis, distortion of the iris, and/or corneal decompensation.

The optical zone portion of the lens in accordance with the present invention may be plano concave or plano convex and may be fabricated from conventional foldable optical materials such as silicone, acrylic, collagen and the like.

The optical zone portion may typically possess any appropriate optical characteristics, for instance, of the corrective type wherein the human crystalline lens is left intact.

Accordingly, the unique phakic IOL and method for implantation for intraocular lens structures avoids the use of sutures which can lead to significant irritation of the ciliary body with attendant difficulty in surgical technique to minimize damage to the iris. However, the inventive haptic design maintains placement of the lens once positioned in the eye, thereby providing a safer and more convenient surgical procedure and a more comfortable fit for the eye.

### BRIEF DESCRIPTION OF THE DRAWINGS

Figure 1 is a partly side sectional view of a human eye for purposes of referencing the description of the unique phakic lens for placement in the anterior chamber in accordance with the present invention;
Figure 2 is a partly side sectional view illustrating one embodied form of the unique phakic lens of the present invention positioned in the anterior chamber of the human eye and further illustrating placement of the lens haptics to rest in the angle of the eye;
Figure 3 is a cross-sectional view of one embodied anterior chamber phakic lens of the plano convex species in accordance with the present invention; and
Figure 4 is a top view of one embodied anterior chamber phakic lens further illustrating the configuration of the abraded haptic members in accordance with the present invention.

### DETAILED DESCRIPTION OF THE PREFERRED EMBODIMENTS

A unique anterior chamber phakic lens and methods for implantation are provided. The foldable optical zone portion of the lens is secured to a pair of diametrically opposed flexible haptic members extending outwardly from the optical zone periphery to the angle of the eye. Each of the haptic members comprises an elongated arm portion and a distal arcuate portion with an inward curvature. The outer surface of the arcuate portion is roughened such as by sanding. Accordingly, the abraded surface of the lens haptics induces a slight amount of scar tissue where the distal ends of lens haptics rest in the angle. The unique phakic lens and implantation methods minimize lens related etiology such as iris chafing, glaucoma, uveitis, distortion of the iris, and/or corneal decompensation.

The inventive lens structures may be utilized for placement of the lens in the anterior chamber of the eye without sutures, for refractive correction. Thus, a safer, more convenient, and more comfortable surgical procedure is achieved with minimized displacement of the intraocular lens structures within the plane of the lens after implantation.

The human crystalline lens is generally recognized to be a transparent structure having a thickness of about 5 millimeters and diameter of about 9 millimeters. The lens is suspended behind the iris by zonular fibers which connect the lens to the ciliary body. A lens capsule surrounds the lens, the front portion of the capsule being commonly known as the anterior capsule and the back portion commonly known as the posterior capsule.

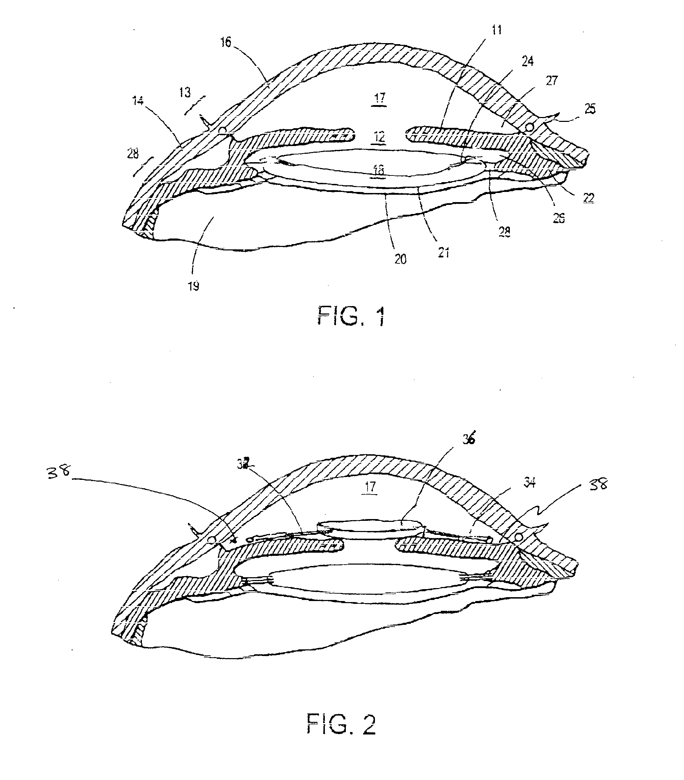

Referring now to the drawing, denoted Figure 1, there is shown a side cross-sectional view of the eye in stylized form illustrating the major ocular components: iris 11, pupil 12, limbus 13 and sclera 14.

In more detail, Figure 1 depicts the cornea 16 composed of clear tissue which connects the sclera 14 at the limbus 13. The anterior segment of the eye is divided into two principle chambers by the iris 11 and pupil 12. The anterior chamber 17 is defined by the space between the cornea 16 and the iris 11. A posterior chamber 18 is defined by the space between the iris 11 and the vitreous 19.

In surgical procedures commonly known as intracapsular cataract extraction, the posterior chamber 18 is bounded by the hyaloid membrane 20. In surgical procedures commonly known as the extracapsular cataract extraction, the posterior chamber 18 is bounded by the posterior capsule 21 attached to the ciliary body 22 by means of zonular fibers 23. Portions of the anterior capsule may remain as flaps 24, creating, with the posterior capsule 21, the ocular portion commonly known as the "capsule bag". The posterior chamber 18 peripheral area between the iris 11 and the extension of the ciliary body 22 is referred to as the ciliary sulcus 26. The anterior chamber peripheral area between the cornea 16 and the iris 11 is referred to as the angle 27 of the eye. The area of the sclera posterior to the plane of the iris and anterior to the vitreous 19 is known as pars plana 28.

With the foregoing referenced ocular components in mind, it is a principle feature of the present invention to provide angle fixation for various classes of phakic intraocular lens structures, with deformable (foldable) optical zone portions such that the lens may be placed within the eye without need for fixating sutures. Accordingly, the placement procedure minimizes the serious dangers associated with UGH syndrome (the triad of uveitis, glaucoma, and hyphema).

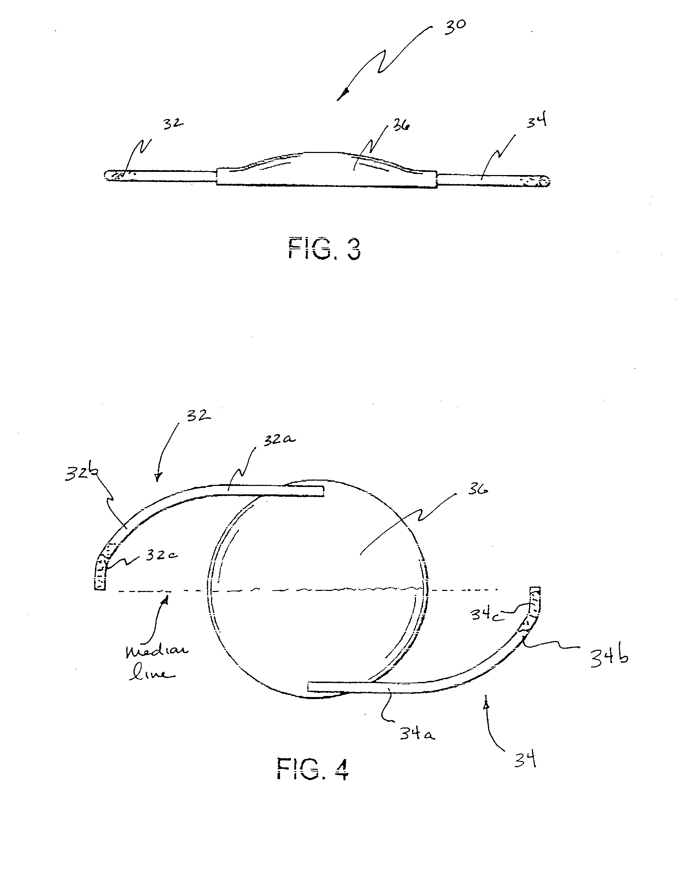

Referring now to the drawing, denoted Figure 2, there is shown one embodied form of the improved phakic intraocular lens structures in accordance with the present invention. In this depicted embodiment, the lens (generally denoted 30) takes the form of a foldable plano convex optical zone portion having an optical axis, wherein the plano surface of the lens faces the natural lens after placement in the eye. A pair of diametrically opposed flexible haptic members 32 and 34 extend outwardly from the periphery of the optical zone portion 36 to one clock hour (about 1 to 4 millimeters) from the angle of the eye. In this respect, the inventive lens is surgically placed in the anterior chamber so that the distal portion of the haptics rests from about 1 to 4 millimeters from the angle interior 38. Each of the haptic members 32 and 34 includes an elongated arm portion 32a and 34a spaced in parallel relation to a median line through said optical zone portion 36, and an distal arcuate portion 32b and 34b with an inward curvature 32c and 34c terminating at the median line through the optical zone portion 36.

The haptics 32 and 34 are appropriately sized to extend no more than one clock hour in angle of the eye (the area between the posterior side of the iris and the ciliary processes) to minimize compression of surrounding ocular tissue.

The optical zone portion 36 will typically have a thickness of from about 0.05 millimeters to about 1.2 millimeters depending upon refractive power, and a diameter in the range of from about 4 millimeters to about 6 millimeters. It is to be clearly understood however, that these dimensions are supplied as merely illustrative of one embodied form of the invention and not restrictive in terms of the dimensions nor configuration of the inventive system.

The deformable optical zone portion 36 is imparted with desirable memory characteristics, appropriate structural dimensions, and composed of a deformable material such that the lens can be deformed to fit through a relatively small size for insertion into the eye, yet return to its original size and fixed focal length after implantation.

One important feature of haptic material, is that it possess the quality of being resiliently rigid but deformable and compliant possessing flexibility. These characteristics facilitate placement of the lens assembly through a relatively small incision made in the ocular tissue or pupil which is relatively smaller than the overall diameter of the lens assembly by deformation, yet allow the lens to return to its full size. and configuration once placed in the eye. Moreover, these qualities make the lens assembly less susceptible to displacement should the lens assembly be subjected to a significant dislocating force. The roughening of the outer surface of the distal haptical portion may be accomplished by any conventional means, e.g., machining, sanding or implanted during molding or during extrusion.

In accordance with the present invention, the optical zone portion of the lens structure may generally be made of foldable materials. In this latter respect, the optical zone portion of the intraocular lens will possess memory characteristics such that the lens can be deformed by compressing, rolling, folding or stretching the optical zone portion to a diameter of 80% or less than the cross-sectional diameter of the optic during insertion into the eye, yet return to its original configuration, size and fixed focal length once imparted in the eye. Typically, the deformable optical zone portion is fabricated from one or more of suitable materials, such as polyurethane elastomer, silicone elastomer, hydrogel polymer, collagen compounds, organic or synthetic gel compounds and combinations thereof.

The intraocular lens structures in accordance with the present invention, are designed for refractive correction without removal of the human crystalline lens. In this respect, the optical zone portion may be of plano-convex, plano-concave or other suitable cross-section for appropriate refractive correction.

Figure 3 is a side sectional view of the intraocular lens assembly depicted in Figure 2, the optical zone portion 36 being the plano-convex type for refractive correction of the human crystalline lens.

Typically, the inventive intraocular lens structures in accordance with the present invention will have a total length of from about 9 millimeters to about 14 millimeters, a width of from about 4 millimeters to about 14 millimeters, and can be fabricated having a wide range of index of refraction. The optical zone portions will typically have a thickness of from about 0.1 millimeters to about 1.0 millimeters and a diameter in the range of from about 4 millimeters to about 6 millimeters.

Any conventional method for manufacture of the inventive lens assemblies can be utilized in accordance with the present invention to insure that the lens has the desired resiliency and compliancy. For instance, compression molding, transfer molding, injection molding, casting, machining, or a combination of these techniques may be utilized to produce the inventive lens assemblies.

The optics of the present IOLs maybe constructed of any suitable material or combination of materials. Such materials include, for example, silicon-based polymeric materials, acrylic polymeric materials, such as PMMA and the like, hydrogel-forming polymers and the like, other polymeric materials, glass and mixtures thereof.

The haptic members can be of various constructions, configurations, and materials. For example, the haptic members may be constructed of PMMA or polypropylene or the like materials. The haptic members are preferably made of polymeric materials. The haptic members can be, and preferably are made of the same material as the optics, and more preferably are unitary and integral with the optics. Preferably, the haptic members are coupled to the optic at generally diametrically opposed locations, for example, secured to the lens body at generally diametrically opposed locations.

In accordance with the present invention, the method for implantation of an artificial intraocular lens for refractive correction of an eye, the method comprises:

The inventive method for implantation of the unique phakic lens for refractive correction of an eye, comprises the steps of: (a) providing a phakic lens having a foldable plano surfaced optical zone portion secured to a pair of diametrically opposed haptic members extending outwardly from the periphery of the optical zone portion to the angle of the eye, the distal portion of the haptic members having a rough exterior surface; (b) deforming the phakic lens by folding the optical zone portion to a diameter of about 80% or less of the cross-sectional diameter of the optic in an unstressed state; (c) inserting the intraocular lens through a relatively small incision made in the ocular tissue for placement in the anterior chamber of the eye; (d) allowing the optical zone portion of the lens to return to its original configuration, full size and fixed focal length wherein the piano surface of the lens faces the natural lens of the eye; (e) effecting placement of the lens haptics to rest in the angle from about 1 to 4 millimeters from the interior wall; whereby the phakic lens creates light attachments in the angle with minimal pressure on the blood vessels in the angle structures.

Accordingly, the phakic lens of the present invention is held in place by the relatively slight pressure from the flexible haptics and the creation of mild fibrosis where the lens haptic rests in the angle. The amount of fibrosis can be effected by roughening the end of the curve.

The unique phakic lens and implantation methods minimize lens related etiology such as iris chafing, glaucoma, uveitis, distortion of the iris, and/or corneal decompensation.

The implantation technique generally uses retrobulbar, peribulbar, or topical anesthesia; a two-plane 6.2 mm posterior corneal incision is centered at 12 o'clock. The first nonpenetrating plane of the incision and the two vertical paracentral paracenteses at 10 and 2 o'clock hours are performed.

The process preferably begins with the creation of a micro (2.7 mm) incision with a diamond knife designed by instant inventor. This incision--the "Blue Line" incision, is safer in terms of closure and heals more rapidly than comparable incisions.

After the intracameral injection of acetylcholine and viscoelastic material, and the second plane of the incision is performed into the anterior chamber. The lens is completely introduced under viscoelastic protection in one step and afterward is rotated 90 degrees into a horizontal position, along the 3 to 9 o'clock direction.

The anterior chamber phakic lens is preferably fixated with a blunt 30G custom-shaped needle provided by Ophtec.

Once the surgery has been completed and the eye has fully healed, vision is remarkably stable. A rich variety of enhancements are possible after the surgery. These enhancements fall into four categories: correction of astigmatism, YAG laser to remove clouding in the capsule after surgery, LASIK to correct near or farsightedness, and though rarely needed, exchange of the intraocular lens itself.

It will be apparent from the foregoing that, while particular forms of the invention have been illustrated and described, various modifications can be made without departing from the spirit and scope of the invention. Accordingly, it is not intended that the invention be limited, except as by the appended claims.

## Claims

1. A phakic intraocular lens for placement relative to the angle in the anterior chamber of the eye, for refractive correction of the natural lens of a patient, the lens comprising:
(a) a foldable optical zone portion having an optical axis and a plano surface, wherein the plano surface faces the natural lens after placement in the eye; and
(b) a pair of diametrically opposed haptic members extending outwardly from the periphery of said optical zone portion to the angle of the eye, each of said haptic members having an elongated arm portion spaced in parallel relation to a median line through said optical zone portion, and a distal arcuate portion with an inward curvature terminating at the median line through said optical zone portion, said distal arcuate portion having a roughened exterior surface.

2. The phakic intraocular lens as defined in Claim 1 wherein said haptic members are configured and sized to fit within one clock hour of the angle in the anterior chamber of the eye.

3. The phakic intraocular lens as defined in Claim 1 wherein said optical zone portion is comprised of a silicone elastomer.

4. The phakic intraocular lens as defined in Claim 1 wherein said optical zone portion is composed of hydrogel polymer.

5. The phakic intraocular lens as defined in Claim 1 wherein said optical zone portion is composed of a collagen compound.

6. The phakic intraocular lens as defined in Claim 1 wherein said optical zone portion is composed of an organic gel compound.

7. The phakic intraocular lens as defined in Claim 1 wherein said optical zone portion is composed of a synthetic gel compound.

8. The phakic intraocular lens as defined in Claim 1 wherein said optical zone portion is composed of acrylic compound.

9. The phakic intraocular lens as defined in Claim 1 wherein the optical zone portion has a plano-convex cross-section.

10. The phakic intraocular lens as defined in Claim 1 wherein the optical zone portion has a plano-concave cross-section.

11. A method for implantation of a phakic intraocular lens for refractive correction of an eye, the method comprising the steps of:
(a) providing a phakic lens having a foldable plano surfaced optical zone portion secured to a pair of diametrically opposed haptic members extending outwardly from the periphery of the optical zone portion to the angle of the eye, the distal portion of the haptic members having a rough exterior surface;
(b) deforming the phakic lens by folding the optical zone portion to a diameter of about 80% or less of the cross-sectional diameter of the optic in an unstressed state;
(c) inserting the intraocular lens through a relatively small incision made in the ocular tissue for placement in the anterior chamber of the eye;
(d) allowing the optical zone portion of the lens to return to its original configuration, full size and fixed focal length wherein the plano surface of the lens faces the natural lens of the eye;
(e) effecting placement of the lens haptics to rest in the angle from about 1 to 4 millimeters from the interior wall, whereby the phakic lens creates light attachments in the angle with minimal pressure on the blood vessels in the angle structures.

12. The method for implantation as defined in claim 9 wherein said intraocular lens is inserted and allowed to return to its original configuration, full size and fixed local length in a position in front of the iris and the pupil of the eye.
